# EUROPEAN PATENT SPECIFICATION

(11) **EP 3 975 956 B1**
(45) Date of publication and mention of the grant of the patent: **05.07.2023**
(21) Application number: 20730764.6
(22) Date of filing: 22.05.2020
(51) Int. Cl.: A61F 13/08

(54) **COMPRESSION STOCKING WITH ADJUSTABLE COMPRESSION PRESSURE**
KOMPRESSIONSSTRUMPF MIT EINSTELLBAREM KOMPRESSIONSDRUCK
BAS DE CONTENTION À PRESSION DE COMPRESSION RÉGLABLE

(30) Priority: 24.05.2019 NL 2023185
(43) Date of publication of application: 06.04.2022
(73) Proprietor: Tobrox Holding B.V., 5581 VL Waarle (NL)
(72) Inventor: STOKBROEKX, Antonius Johannes Cornelis, 5581VL Waalre (NL)
(74) Representative: Arnold & Siedsma
(86) International application number: PCT/NL2020/050331
(87) International publication number: WO 2020/242297

(56) References cited:
- WO-A1-2018/052676
- WO-A2-2012/142155
- DE-A1-102014 013 392
- GB-A- 319 936
- US-A1- 2014 094 726

## Description

The invention relates to a compression stocking with adjustable compression pressure for therapeutic compression of a limb, particularly a leg.

Chronic venous insufficiency (CVI) is a condition of the (lower) limbs. The condition is related to an insufficiency (i.e. a functional or physical defect) of one or more veins in the limbs. The insufficiency is usually caused by failure or impaired function of the venous valves. The most extreme form of CVI symptoms is a venous (leg) ulcer. For treatment of such a venous insufficiency, and also for other venous or arterial problems for that matter, use is often made of compression therapy. This involves arranging an elastic stocking around the limb which is causing complaints, or wrapping the limb in bandages. The elastic compression stocking fits very tightly round the limb so as to be able to exert a sufficiently great compression thereon. Bandages must be pulled very tight in order to be able to exert sufficient compression on the limb. For older patients in particular it is often no longer possible, especially in higher compression classes, to pull on these compression stockings themselves or to tighten these bandages sufficiently themselves. A nurse or similar carer will have to step in to put on and take off the stockings or wrap the bandages for the patient.

Because the compression therapy is only effective when sufficient compression is exerted on the limb, the degree of compression must be monitored periodically (up to a few times a day). If the compression is too low, a new stocking must be put on or the limb must be re-bandaged. This is an inefficient and expensive activity when having to be done by a carer. Even if the patient is able to do it themselves, it puts a lot of strain on him or her. There is therefore a need for a support stocking which can be put on more easily, without a carer being needed to do so.

A further drawback of the known elastic stocking or the known bandages is that the degree of compression is inaccurate. It can in fact not be measured, or hardly so, which compression (pressure) is being exerted on the limb. This also makes it difficult to measure the effect of the compression therapy.

The distribution of the compression pressure over the surface of the limb is further inaccurate. What happens here is that after some time, for instance after a few hours, the pressure exerted on the limb has decreased because the limb has decreased in size. The support stocking or bandages can then become loose locally, which can diminish the therapeutic effect.

Support stockings wherein use is made of an adjustable compression pressure are per se known in the field. In a determined type of support stocking with adjustable compression the stocking is provided with an inflatable pressure chamber whereby the compression exerted on the limb can be varied by inflating the pressure chamber. Examples of such a type of support stocking are described in patents WO 03/007855 A1 and DE 10 2008 029 289 A1. Such an inflatable support stocking is per se advantageous because it is much simpler to put on the support stocking, since inflation need not be carried out until after the stocking has been put on. A further advantage is that the pressure (compression) is controllable in principle. A further advantage is that, if the limb were to shrink at a certain point and the support stocking were thereby to become looser, the stocking can be made tighter again in simple manner by additionally inflating the pressure chamber.

The known support stockings however do have a number of drawbacks. A drawback is for instance that, as a result of the very low overpressures which are generated in the stocking (characteristically 30-60 mm Hg), it is difficult to have a properly functioning valve mechanism (valve) with which the loss of air pressure is limited to a minimum or is avoided over the course of time (for instance during the course of the day). A further drawback is that the known valves whereby the support stocking is connected to the outside world are often complex and/or protrude too far outside the stocking, this making them inconvenient for the patient and clothing.

Known from WO 2012/142155 A2 is a therapeutic compression stocking consisting of a first stocking element (calf wrap) which is arranged around the lower leg of a patient and a second stocking element (foot wrap) which is arranged around the foot of the patient. Provided in both stocking elements is a pressure compartment (bladder) which can be inflated with air.

The pressure compartment however has limited dimensions and extends only at the position of the calf (i.e. the rear side of the lower leg) and the heel (i.e. the underside of the foot). No pressure compartment is present at the position of the other parts of the leg with which the compression stocking is in contact, for instance the front side of the lower leg and the sides of the foot. A different pressure will be exerted on the leg at positions where no pressure compartment is present than on parts where the pressure compartment is present. The known compression stocking therefore does not exert a uniform pressure. This can greatly reduce the effect of the compression stocking and/or the comfort for the patient.

The fact that a pressure compartment is not present over the whole contact surface of the compression stocking with the lower leg, and variations in compression thus occur over the contact surface, is not the only reason for the reduced effect of the stocking and/or degree of comfort for the patient. These drawbacks occur all the more because the known compression stocking is further provided with a large number of through-holes which form an open connection between the outside air and the leg. No compression of the leg whatsoever thus occurs in the large number of areas where such a through-hole is present. The effectiveness of the known compression stocking at preventing the above stated complaints is therefore limited.

It is an object to provide an improved compression stocking wherein at least one of the above stated drawbacks is obviated.

It is also an object to provide a compression stocking which has such a simple construction and/or can be manufactured in such cost-effective manner that it can be replaced with a new support stocking after one use or only a limited number of (for instance two or three) uses.

It is also an object to provide a compression stocking which enables an improved compression therapy and/or can increase comfort for the user.

At least one of the objectives is achieved in a compression stocking with adjustable compression pressure for therapeutic compression of a leg, the compression stocking comprising:
- a first stocking element to be arranged around the lower leg;
- a second stocking element to be arranged around the foot of the leg, wherein the second stocking element is attached to the first stocking element or formed integrally thereon at an angle;

further comprising a pressure compartment to be filled with air up to the adjustable compression pressure and extending in both the first and the second stocking element, and configured to extend all the way around the periphery of at least a part of the lower leg in use in order to exert a homogenous pressure on the at least one part of the lower leg all the way around;
a valve mechanism connected to the pressure compartment, wherein the valve mechanism preferably comprises a closable passage between the pressure compartment and the outside air, wherein the valve mechanism comprises:
   - a valve seat attached to the stocking element;
   - a valve lid attached to the valve seat or formed thereon, wherein the passage extends through the valve seat and the valve lid;
   - a valve provided in the passage and embodied to be movable between a closing position, in which the valve closes the passage airtightly, and a position allowing passage, in which the valve leaves the passage at least partially open; and
   - at least a connecting part between the valve and the valve seat and/or the valve lid for flexible attachment of the valve to the valve seat and/or the valve lid, wherein the at least one connecting part is embodied to urge the valve toward the closing position under bias.

The pressure compartment can further be embodied to wholly surround the lower leg in peripheral direction in the situation in which the stocking is being worn so that the pressure compartment extends over the whole contact surface (whole in the sense of the peripheral direction of the lower leg in question) of the stocking with the lower leg, and the lower leg encounters an identical pressure over this whole contact surface (since the pressure is the same in the whole pressure compartment, in accordance with Pascal's law). What this amounts to is that the pressure compartment at the position of the first stocking element extends on the front side, the rear side and on the sides of the lower leg during use. The compression pressure is adjustable by controlling the amount of air introduced into the pressure compartment, but is constant over the whole periphery of the (part of the) leg. This has a positive effect on the uniform compression on the leg, and so on the effect of the compression stocking. The patient further feels no pressure differences over the surface of his or her lower leg, which improves comfort for the patient. In order to exert the correct compression pressure on the foot said pressure compartment extends into the second stocking element, this such that the pressure compartment is situated only on the sides of the foot during use. The pressure compartment has been dispensed with at the position of the sole of the foot and the instep of the foot. By exerting the set compression pressure only on the sides of the foot a superior operation of the compression stocking can be realized. The patient is further able to walk properly with the compression stocking because no pressure compartment is present under the sole and/or the compression stocking is compact enough for shoes or slippers to still be worn over it.

In determined embodiments the pressure compartment in the first and second stocking element can further be located over the whole surface of the stocking intended for contact with the leg, with the exception of the surface of the sole and instep. The pressure is thereby homogenous on the leg at every position where the stocking is present.

According to further embodiments, the pressure compartment forms one single, uninterrupted space. In determined embodiments there are further no passages such as through-holes between the outer and inner side of the stocking and the like. A uniform distribution of the compressive forces over the contact surface of the support stocking directed toward the lower leg is hereby achieved. The pressure space is further uninterrupted. There are no surfaces present in the contact surface with the lower leg directed toward the lower leg where less pressure or no pressure (i.e. only the ambient air pressure) is exerted on the lower leg (or the foot).

In respect of the valve mechanism, it is the case that the provided bias is preferably greater than the force which is exerted on the valve as a result of the pressure difference between the air pressure in the pressure compartment and the outside air pressure. This ensures that the valve always tends to move toward the closing position in order to close the passage airtightly. This means that the compression pressure remains constant (at the correct pressure value) and does not tend to decrease to greater or lesser extent during and after removal of the air pump with which the compression pressure is realized. This improves the operation of the compression stocking.

In practice said pressure difference amounts to a maximum of about 80 mm Hg, wherein the more usual pressures lie slightly lower, for instance in the order of magnitude of 30 to 60 mm Hg. These pressures are so low that in known valves without said connecting part they will often be insufficient to make the valve mechanism close. In practice the valve is then sometimes not closed properly, so that air can escape from the pressure compartment and the compression on the limb will become too low at a given moment. In the compression stocking as described here it is thus the connecting part and not primarily the air pressure difference between the pressure inside the pressure compartment and the outside air that ensures that the valve mechanism tends to close.

In an embodiment the second stocking element comprises:
- a sole part;
- an instep part;
- side parts between the sole part and the instep part, wherein the pressure compartment extends in at least one of the side parts.

The sole part, instep part and side parts are preferably embodied to be situated under, above and to the side of the foot of the leg in a situation of use. In a further embodiment a pressure compartment is arranged neither in the instep part nor in the sole part. The sole part is used for walking on, and a good contact must be maintained between sole and ground surface. The sole part is preferably manufactured from non-elastic material. This has the result that the one or more pressure compartments of the compression stocking are positioned accurately relative to the different components of the foot in order to be able to exert pressure on the foot in precisely the right area.

It has for instance been found by the inventor that it is particularly exerting pressure on *malleolus lateralis* (i.e. the piece of bone (prominence) on the outer side of the leg/the foot, formed by the lower outer end of the fibula protruding laterally outward), optionally also exerting pressure on *malleolus medialis* (i.e. the piece of bone (prominence) on the inner side of the leg/the foot, formed by the lower outer end of the tibia), that produces good results in the treatment of chronic venous insufficiencies (CVI). In preferred embodiments the at least one pressure compartment is therefore located at least adjacently of the *malleolus lateralis*, preferably also adjacently of the *malleolus medialis*, in a situation of use. Correct dimensioning of the non-elastic sole part enables the pressure compartment to be positioned relative to the foot with such accuracy that the support stocking presses on the *malleolus* exactly at the right position.

In further embodiments the instep part is embodied in elastic material. The elastic embodiment enables the compression stocking to already be clamped fixedly to the foot to some extent, which facilitates putting on of the stocking.

In a preferred embodiment the second stocking part is embodied to be slidable directly onto the foot, and cannot be opened. The first stocking part can be opened, and can thereby be easily arranged round the lower leg (calf) in fully opened position. The edges of the first stocking part are then fastened to each other, and inflation of the pressure compartment can commence.

In determined embodiments the compression stocking has only one single pressure compartment. This has the advantage, among others, that the whole stocking can be inflated via a single valve mechanism and/or that the pressure in the pressure compartment is uniform over the whole stocking. In other embodiments the compression stocking has two or more optionally separated pressure compartments. These pressure compartments are then however in open connection with each other so that the pressure is the same in all pressure compartments, and the same pressure is thus exerted at the position of the pressure compartments over the whole surface that presses against the leg.

The pressure compartments can be operated (inflated and deflated) by one single, collective valve mechanism. In further embodiments the pressure compartments however each have their own valve mechanism.

In an embodiment the at least one connecting part is embodied to be bent between a starting position, in which the valve is in the closing position, and a bent position, in which the valve is in the position allowing passage, under an external mechanical force. The valve mechanism is here preferably embodied such that the valve is only opened under the influence of the mechanical force. The mechanical force can for instance be produced by a pressing force which is exerted on the valve by the outer end of a pump tube or conduit inserted into the passage. Additional forces, such as an increased air pressure, are thus unnecessary. The connecting part is further embodied such that in the bent position it tends to return to the starting position (in which the valve closes the passage). In a determined embodiment the connecting part is manufactured from elastic material, at least material sufficiently bendable under external force. When the external force is released, for instance because the pump is detached from the valve mechanism, the valve returns to its starting position.

In a determined embodiment the compression stocking comprises two or more connecting parts, wherein the valve is disc-like, the connecting parts extend in radial direction relative to the disc-like valve and/or the connecting parts are attached to or formed on the valve at uniformly distributed positions.

As already stated above, the external force on the valve can be produced by the outer end of a conduit or the like of a pump with which the pressure compartment can be inflated. In further embodiments an air pump is provided which is configured to inflate the pressure compartment. The air pump comprises a pump tube or pump conduit which can be coupled to the valve lid. The pump tube or pump conduit has a (free) outer end which can be inserted into the passage and is embodied to exert in inserted situation the above stated mechanical force whereby the valve can be pushed from the closing position to the position allowing passage. In determined embodiments the outer end can be clamped fixedly on or otherwise secured in said passage. The outer end is preferably clamped fixedly such that the clamping force is greater than the force exerted by the at least one connecting part on the valve in order to urge the valve back to its starting position. The valve hereby remains opened until the outer end has been removed from the passage again.

In determined embodiments a pressure sensor is provided for the purpose of determining a pressure value representative of the pressure in the pressure compartment. The pressure sensor can here be a permanent pressure sensor and/or can be embodied to measure the pressure inside the pressure compartment. The pressure sensor can be an internal pressure sensor which is arranged inside the pressure compartment, for instance integrated with the valve mechanism, but can also be an external pressure sensor which is mounted on the outer side of the pressure compartment and is in pressure connection therewith. In other embodiments the pressure sensor does not form part of the support stocking but is for instance a separate pressure sensor or a pressure sensor which forms part of the air pump. In the latter case the pressure sensor can be embodied to measure the pressure in the pump tube or pump conduit. This pressure measured in the conduit or tube is directly or indirectly representative of the pressure in the pressure compartment.

In determined embodiments, wherein the pressure sensor is provided with a display on which the pressure is displayed or wherein the pressure can be shown in other manner, the person inflating the pressure compartment can keep an eye on the pressure during the inflating. The pressure sensor (optionally in combination with a control unit) can be set to generate a visual and/or auditive warning signal at a desired pressure to be set by the therapist. A coloured light signal (for instance via said display and/or via one or more optionally coloured lights, for instance red and green LED lamps) can for instance be generated when the pressure is too low, too high or correct. When the pressure has reached a predetermined desired value, he or she will stop the inflating and remove the pump from the support stocking. The valve will close automatically, and the support stocking is ready for use. In other embodiments this process can be automated. In a preferred embodiment the support stocking and/or the air pump comprises a control unit which is coupled to the air pump and pressure sensor and is configured to control the air pump depending on the measured pressure. The control unit can for instance be configured to increase the pressure in the pressure compartment until a preset maximal pressure value, for instance stored in the control unit or in a separate memory, is reached.

Provided in determined embodiments is a memory which is coupled to the pressure sensor and is configured to periodically store measured pressure values. The therapy adherence of the patient can for instance be measured hereby.

In determined embodiments, for instance - though not limited to - embodiments in which the pressure sensor is placed inside the pressure compartment, the pressure sensor comprises a wireless communication unit for wirelessly transmitting a pressure signal representative of the pressure in the pressure compartment. The pressure signal can here be representative of the current pressure measured by the pressure sensor and/or of one or more of the measured pressure signals from the memory coupled to the pressure sensor.

In a further embodiment the communication unit, and preferably also the pressure sensor and/or the control unit, is configured to be powered only by an external radio frequency signal. They do not have their own power source or energy source, such as a passive tag or an RFID transponder. Such a communication unit can utilize the electromagnetic field of an external reader to induce a current in a coil, with which (a chip in) the communication unit is powered.

In order to achieve a good compression on the limb, keep the external dimensions of the support stocking in inflated state limited yet still enable a high degree of comfort for the patient, the compression stocking comprises according to determined embodiments:
- an outer wall with a high modulus of elasticity (E), preferably a modulus of elasticity of at least 50 MPa.

The compression stocking preferably comprises an inner wall, directed toward the limb during use, of a flexible material which closely follows the contour of the limb.

In a further embodiment the stocking element comprises an outer wall with an elasticity (ε) of a maximum of 5%, preferably a maximum of 2%, when the pressure compartment is inflated to an overpressure of 100 mg Hg. With such an outer wall sufficient compression can be realized without the outer dimensions of the support stocking becoming too great, which would reduce the wearer comfort of the support stocking.

In a determined embodiment the stocking element comprises an outer and inner wall manufactured from airtight film, wherein the outer and inner wall are preferably sealed together along their peripheral edges for the purpose of forming the pressure compartment therebetween. In this way a comfortable support stocking can be realized quickly and at relatively low cost.

It is otherwise usual also to arrange an additional layer between the compression stocking and the limb. In the case of an ulcer, bandaging is worn underneath the compression stocking. In other cases, a desired thin stocking can be worn underneath it.

The material of the inner wall (for instance TPU film) of the compression stocking is preferably chosen such that it does not feel unpleasant on the skin per se.

In determined embodiments the stocking element of the support stocking comprises an outer and inner wall manufactured from plastic, particularly thermoplastic polyurethane or polypropylene. The outer wall preferably has a wall thickness of between 100 and 300 µm, and the inner wall a wall thickness of between 50 and 100 µm. This wall thickness for the outer wall provides a good compromise between flexibility and minimal stretch when a TPU film is used. The inner wall should be as thin as possible, while maintaining airtightness and applicability in chosen manufacturing techniques.

In determined embodiments the outer wall of the stocking element is provided with a number of stretch-limiting elements configured to limit stretch when the pressure compartment is inflated. A stretch-limiting element can for instance comprise a strengthening thread arranged in or on the outer wall. These are oriented such that stretch in a determined direction can be limited. The strengthening threads can for instance extend in peripheral direction when the stocking element is arranged around the limb. When the stocking element extends vertically, the strengthening threads thus extend in horizontal direction (i.e. the width direction). Expansion in radial directions is limited hereby, so that the dimensions of the support stocking in those directions also remain limited.

Further advantages, features and details will be elucidated with reference to the following description of some embodiments thereof. Reference is made in the description to the accompanying figures, in which:
- figure 1 shows a view of a leg provided with the embodiment of a support stocking according to the invention;
- figure 2 shows a view of the embodiment of the support stocking of figure 1 before it is arranged round the leg;
- figure 3 shows a perspective detail view of a valve mechanism according to a determined embodiment;
- figure 4 shows a perspective exploded view of the valve mechanism of figure 3;
- figure 5 shows a cross-section through the valve mechanism of figure 4;
- figure 6 shows a cross-section through the embodiment of the support stocking of figure 1, in combination with a pump for inflating the support stocking, in a situation before the pressure compartment is inflated;
- figure 7 shows the cross-section of figure 6 in a situation during inflation;
- figures 8 and 9 show cross-sections through another embodiment, respectively in the situation before and during inflation, wherein the support stocking is provided with an internal pressure sensor;
- figure 10 shows a top view of the internal pressure sensor of figures 8 and 9;
- figure 11 shows a perspective front view of a further embodiment of a stocking according to the invention, with a first stocking element in opened state;
- figure 12 shows a side view of the embodiment of figure 11 in closed situation; and
- figure 13 shows a side view of a leg provided with a stocking according to the embodiment of figures 11 and 12 in a situation of use.

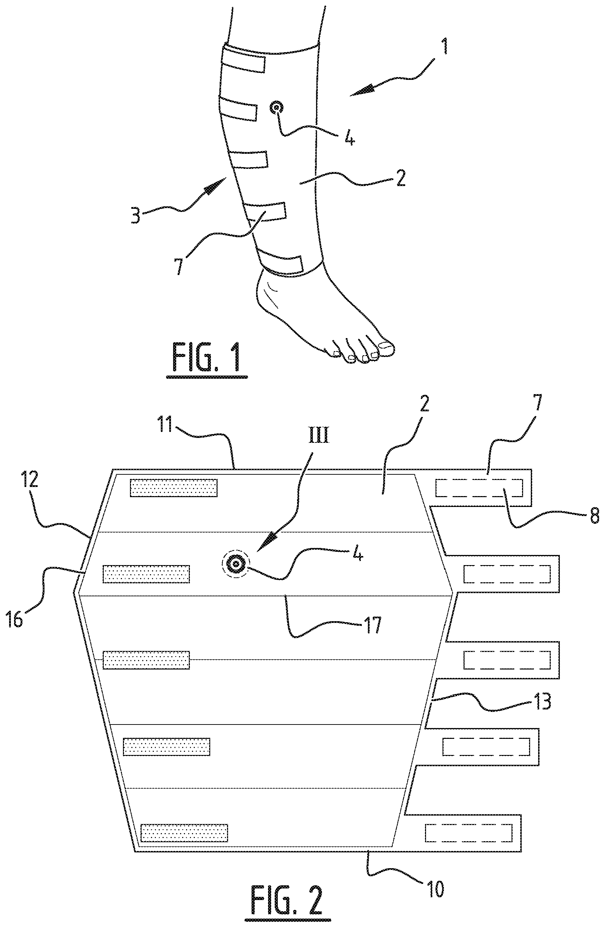

Figure 1 shows an embodiment of a support stocking 1 in a situation of use. This embodiment of the stocking covers only the calf of a patient. In other embodiments the foot and/or the upper leg of the patient can however also be covered. In other embodiments the support stocking is arranged round an arm or the like. Support stocking 1 comprises a stocking element 2 which is clamped fixedly around the limb using fastening means 3. Figure 2 shows the fastening means 3 in more detail.

In the shown embodiment the stocking element 2 is constructed from a multi-layer film, wherein a pressure chamber or pressure compartment is formed between the layers of the multi-layer film. The multi-layer film can be wrapped around the limb and then be secured, particularly be clamped fixedly, in wrapped state, for instance by attaching a longitudinal edge of the support stocking to another part of the support stocking with fastening means.

In the shown embodiment fastening means 3 comprise a number of (i.e. in the shown example five, although this number can vary) fastening tongues 7 which are provided on one side with a strip of hook and loop elements 8. Tongues 7 are provided along a longitudinal edge 13 of stocking element 2. A number of hook and loop elements is arranged on the opposite longitudinal edge 12 of stocking element 2, at positions corresponding with those of tongues 7. Once stocking element 2 has been wrapped around the leg, it can be clamped fixedly to the leg using the hook and loop elements 5, 6 so that the support stocking remains at the desired position. The support stocking need however not be arranged with such force that the therapeutic compression pressure is realized.

The therapeutic compression pressure is however realized by inflating the pressure compartment of the support stocking. As already explained above, stocking element 2 comprises a number of layers of film, between which a pressure compartment is formed. Stocking element 2 of the support stocking can for instance be constructed from an outer layer or outer wall 14 and an inner layer or inner wall 15. The outer and inner wall 14, 15 are attached to each other at their peripheral edges (for instance by welding (sealing) them together, which results in the welded seam 16 shown in figure 2, which extends along the upper side 11, lower side 10 and longitudinal edges 12, 13). In this way a pressure compartment 9 is realized between the outer and inner wall 14, 15. This pressure compartment 9 can be provided with air from the outside using a valve mechanism 4 in order to generate a sufficiently high compression pressure in pressure compartment 9 and thus allow the compression stocking to push with sufficient compression pressure against the limb.

The compression caused by the pressure in the pressure compartment of the first stocking element and the second stocking element is the same everywhere. This provides for a uniform distribution of the pressure over the whole contact surface of the stocking with the leg, at least at the positions where the pressure compartment is situated. Since the pressure compartment is situated around at least a part of the lower leg, so both on the front side and on the rear side thereof, and at the position of the sides of the foot, the pressure and thereby the compression caused will be the same everywhere in those areas. The patient experiences the same pressure on all sides of the lower leg (i.e. front side, rear side and both sides), which makes the stocking extremely comfortable.

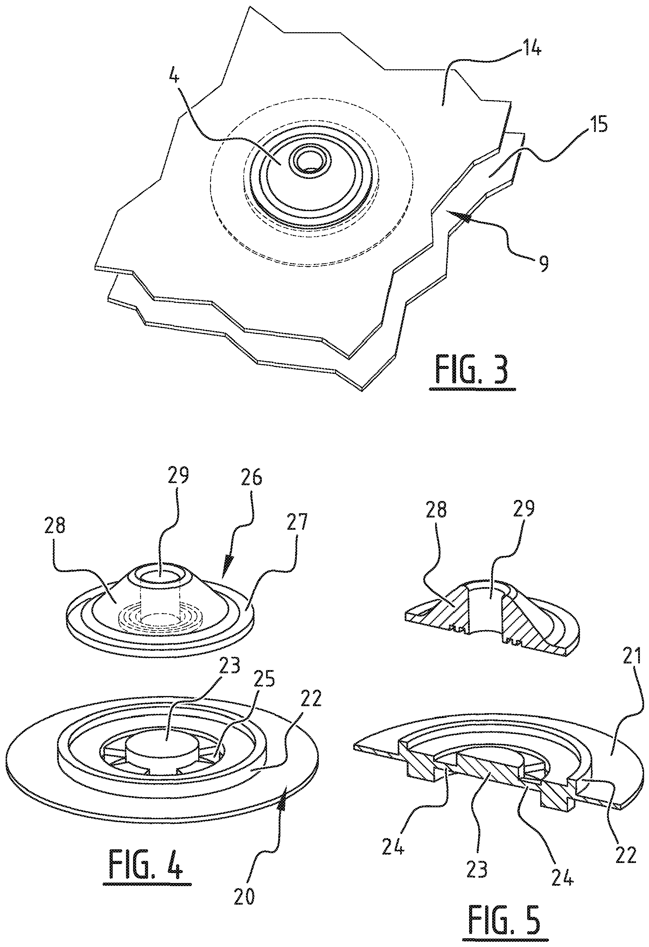

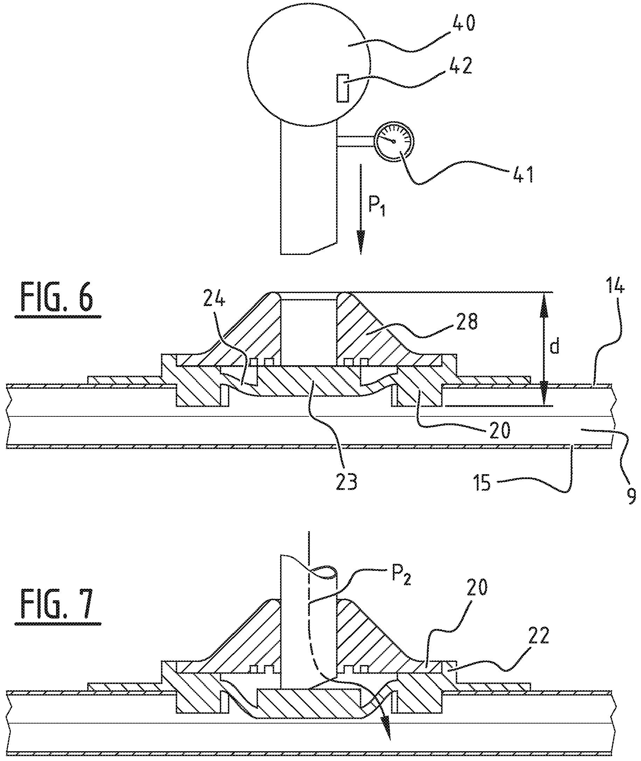

Referring to figures 3-5, a first embodiment of valve mechanism 4 is first described. Valve mechanism 4 comprises a valve seat 20 attached to outer wall 14 by means of ultrasonic welding. This valve seat 20 comprises a radial flange 21 which provides for a sufficient attaching option of the valve mechanism to outer wall 14. Radial flange 21 is further provided with an upright, axially extending edge 22 around an opening 25. Positioned centrally in this opening 25 is a disc-like valve 23, which valve 23 is held in place by a number of (in the shown embodiment four) connecting parts 24. These connecting parts extend in radial direction from the centre of valve 23 and form as it were a star-shaped attachment of valve 23 to the rest of valve seat 20. Valve mechanism 4 further comprises a valve lid 26 attached to the valve seat or formed thereon. This valve lid 26 can be slid between upright edge 22 and be clamped fixedly onto valve seat 20. This situation is shown in figure 6. The valve seat and the valve lid lie tightly against each other such that essentially no air can flow via the valve lid and the valve seat into and out of compartment 9, besides via the passage 29 provided centrally in valve lid 26. The passage 29 and the valve lid connect to valve 23, this such that in the starting position (with the valve in the closing position, as shown in figure 6) valve 23 fully closes the passage 29 so that no air is able to flow into the pressure compartment or out of the pressure compartment.

Figure 6 further shows how the pressure compartment 9 of the support stocking can be inflated. Use is made for this purpose of the schematically shown pump 40. This can be any pump, for instance - though not limited to - a diaphragm pump 40. Pump 40 comprises a pump conduit or pump tube 30 and a pressure sensor 41 connected thereto, with which the pressure inside the pump conduit or tube can be measured.

The conduit or tube 30 has a shape and dimensions such that it fits just inside the passage 29 in valve mechanism 4. When the air compartment has to be inflated, the outer end of the pump tube/pump conduit 30 is displaced in the direction of valve mechanism 4 (direction P₁), and the outer end thereof is inserted fittingly into passage 29. At a given moment the outer end of the pump tube/pump conduit 30 will press onto the upper side of valve 23 and, when pushed further (direction P₁), the valve will be pressed downward by the downward mechanical force caused thereby (which is greater than the upward force exerted on valve 23 by the connecting parts), as is shown in figure 7. In the bent position of connecting parts 24 shown in figure 7 the connecting parts will exert an upward force on valve 23 and will try to push valve 23 back to the starting position shown in figure 6. The external mechanical force is however greater, and valve 23 remains in the position allowing passage which is shown in figure 7. Valve 23 then no longer closes the underside of passage 29. Air can then flow via the pump tube/pump conduit 30 (direction P₂) and the opening between connecting parts 24 into pressure compartment 9.

Inflation of the pressure compartment can be done manually, but can also be controlled by a control unit 42 coupled to pump 40 and pressure sensor 41 (figure 6). When the pressure gauge or pressure sensor 41 measures the desired final pressure, it transmits a signal to control unit 42. Control unit 42 stops inflation and the conduit/tube 30 can be pulled out of the passage again. As a result of the construction of the connecting parts they will return from the bent position shown in figure 7 to the starting position shown in figure 6, so that the valve is returned from the position allowing passage to the closing position.

Pressure sensor 41 can also be used for randomly or periodically monitoring the pressure afterwards, for instance after decreasing due to reduced swelling. If the pressure has become too low, control unit 42 generates a signal representative of the fact that the pressure has become too low, on the basis of which a visual and/or auditive warning signal can be emitted and the patient is informed that the stocking should be refilled with air.

The shown construction ensures that when pump conduit 30 is removed from passage 29, valve 23 always ends up back in the starting position (i.e. the closing position) automatically as a result of the forces exerted by the connecting parts 24. The construction of the valve mechanism is further very simple and can be manufactured in large numbers and at relatively low cost. A further advantage is that the overall thickness of the valve mechanism can remain limited. In the shown embodiment the thickness (d) (see figure 6) of the valve mechanism is for instance a maximum of 1 cm, preferably smaller than 0.5 cm.

Figure 2 further shows that a number of stretch-limiting elements 17 is arranged in and/or on the material of the stocking element, for instance in the material of outer wall 14 thereof. When the stocking element is arranged around a limb, the stretch-limiting elements 17 extend in the peripheral direction. These stretch-limiting elements are less elastic (i.e. have a lower elasticity (ε), defined as the ratio between the change in length and the original length of a piece of material) than the material itself, and thus provide for a limitation of the stretch in the direction in which the stretch-limiting elements extend (therefore only in the width direction in the shown embodiment).

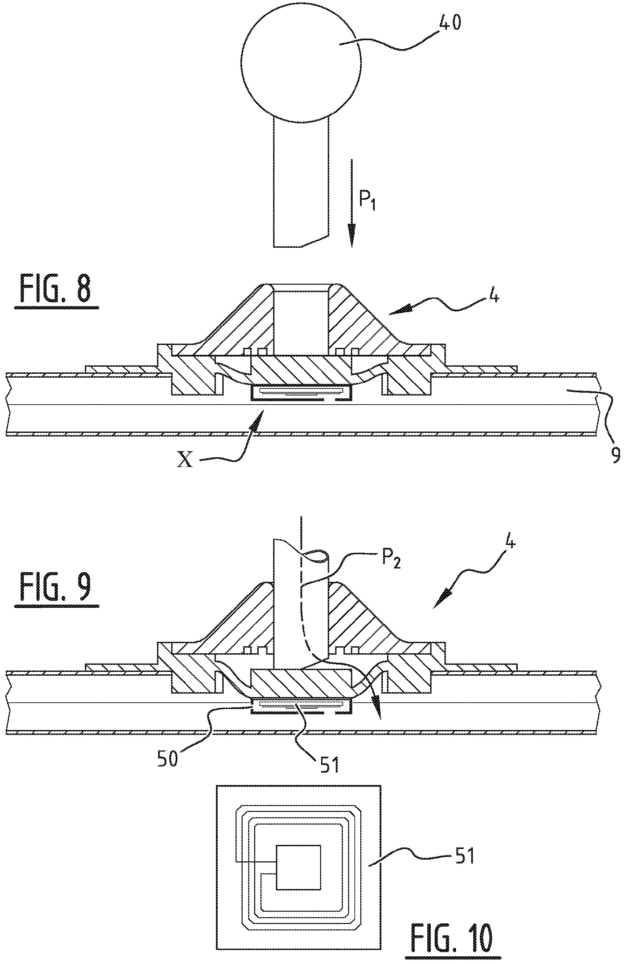

In the embodiment shown in figures 6 and 7 the pressure sensor 41 is provided as part of pump 40. In the other embodiment, for instance the embodiment as shown in figures 8 and 9, pressure sensor 50 is arranged inside pressure compartment 9. More particularly, pressure sensor 50 is integrated with valve mechanism 4, for instance by mounting the pressure sensor on the underside of valve 23. In order to ensure that the pressure measured by the sensor can be easily read, the sensor can be provided with a wireless communication unit 51 for wirelessly transmitting a pressure signal representative of the pressure in a supply compartment. This can be the current pressure measured by the pressure sensor at that moment. In the other embodiments, the pressure sensor is however provided with a memory (not shown in the drawings) in which pressure values are occasionally (periodically) stored. These pressure values are thus stored and can be collectively transmitted via a pressure signal to an external receiver (not shown) using the wireless communication unit.

Pressure sensor 50 (optionally provided with a memory and optionally provided with a communication unit) is preferably embodied as a passive element, i.e. this sensor does not have its own energy source as power supply but is powered by the electromagnetic radiation, for instance electromagnetic radiation from the receiver. More particularly, use can be made of an RFID tag as shown in figure 10, whereby information from the pressure sensor can be transmitted wirelessly to an external RFID receiver.

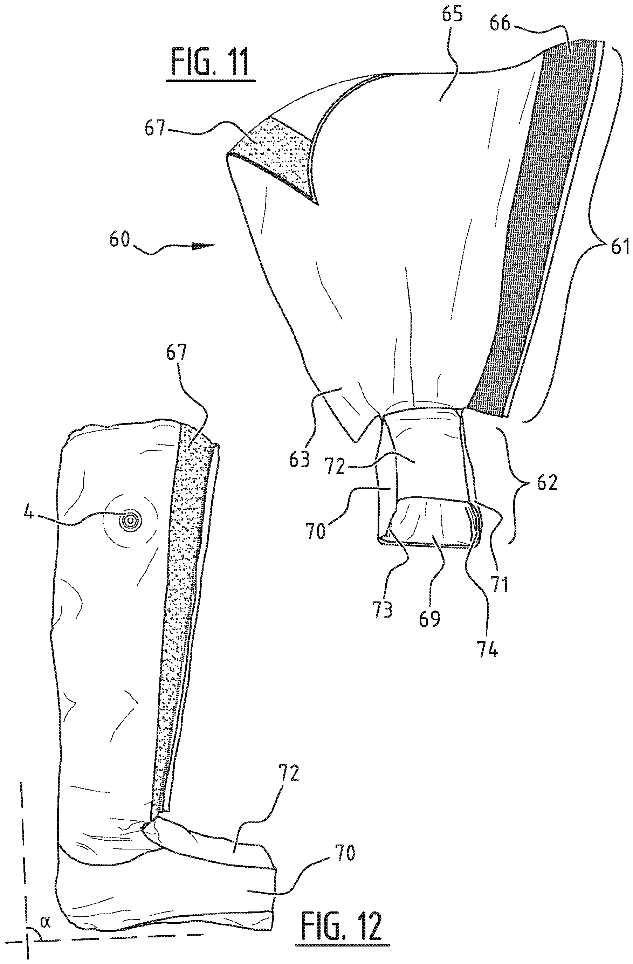

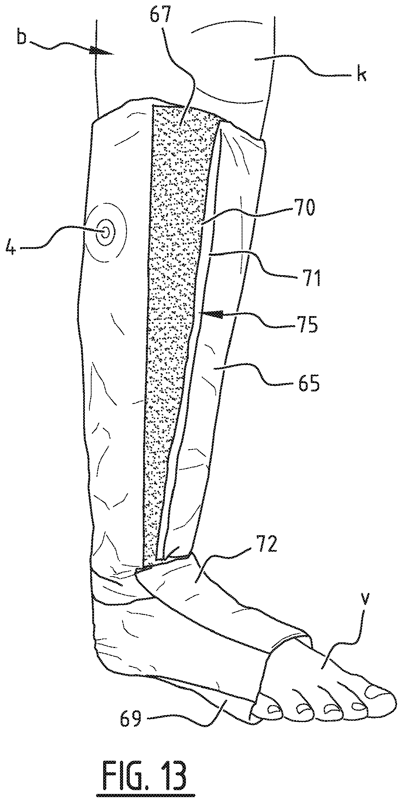

A further embodiment is shown in figures 11-13. The figures show a stocking 60 comprising a first (upper) stocking element 61 and a second (lower) stocking element 62 formed thereon. The first stocking element 61 is embodied to be arranged around the calf (k) of a leg (b) (figure 13), while the second stocking element 62 is embodied to be arranged around the foot (v) of the leg (b). A single pressure compartment 63 is arranged in the stocking. Pressure compartment 63 extends in both the first and the second pressure element 61, 62. More particularly, pressure compartment 63 comprises a first pressure compartment part 75 in first stocking element 61 and two pressure compartment parts 73, 74 in second stocking element 62. All pressure compartment parts are in open connection with each other and can be filled with air via a valve mechanism, preferably a valve mechanism 4 as described above.

Second stocking element 62 is attached to or formed on first stocking element 61 at an angle (i.e. angle α, figure 12, preferably in the range of 60° < α < 120°) such that the stocking can be arranged comfortably around the leg and can fulfil its compressing function.

In the shown embodiment second stocking element 62 comprises a sole part 69, an instep part 72 and at least two side parts 70, 71 between the sole part and the instep part. As shown in figure 13, sole part 69, instep part 72 and side parts 70, 71 are embodied to be situated respectively under, above and to the side of the foot (v) of the leg in a situation of use. In determined embodiments at least one of the sole part and the instep part is manufactured from elastic material so that the stocking can be slid over the foot and already remains clampingly on the foot to a certain degree. In order to bring about the correct (higher) degree of compression on the foot the pressure in pressure compartment parts 73, 74 can be increased as desired by supplying air via valve mechanism 4. This is because pressure compartment 63 of stocking 60 extends in at least one of the side parts (preferably in both side parts 70, 71) and each of the pressure compartment parts 73, 74 inside second stocking element 62 is in (open) connection with the pressure compartment part 75 inside first stocking element 61.

First stocking element 61 is constructed from at least an inner wall 70 and an outer wall 71. More generally, both walls 70, 71 are manufactured from flexible and pliable material so that stocking element 61 can be wrapped easily round a leg of a random user. The walls 70, 71 of first stocking element 61 and the walls of the side parts of second stocking element 62 preferably further take an airtight form and are attached (for instance sealed) to each other along the edges such that the pressure compartment 63 is formed between the two walls 70, 71.

The material of the walls 70, 71 of first stocking element 61 and that of side parts 70, 71 of the second stocking element are preferably manufactured from plastic, such as thermoplastic polyurethane (TPU) or polypropylene. The outer walls preferably have a wall thickness of between 100 and 300 µm and the inner walls a wall thickness of between 50 and 100 µm.

When the user wishes to put on a stocking 60, he or she places a foot in the second stocking element 62 and ensures that second stocking element 62 is held tightly around the foot as a result of the elastic material properties of instep part 72 (and/or sole part 69). The user then closes the first, opened stocking element 61 from the opened position shown in figure 11 and fastens the longitudinal edges of first stocking element 61 to each other by means of suitable fastening means. In the shown embodiment the fastening means comprise a first strip of hook and loop element 66 (provided on a first longitudinal outer end of stocking element 61) and a second strip of hook and loop element 67 (provided on a second, opposite longitudinal outer end of stocking element 61). When the two strips are pressed together, first stocking element 61 can be attached to the leg, essentially independently of the shape and dimensions of the calf. A suitable medium (for instance a gas such as air) can then be supplied via valve mechanism 4 so that the pressure compartment in first and second stocking element 61, 62 expands and thereby allows the stocking to exert the desired, adjustable compression force on both the calf and the foot.

Although not shown in the figures, in the embodiment of figures 11-13 the material can also be provided with one or more stretch-limiting elements, for instance stretch-limiting means of the above described type.

The invention is not limited to the embodiment thereof described herein. The rights are defined by the following claims, within the scope of which many modifications can be envisaged.

## Claims

1. Compression stocking with adjustable compression pressure for therapeutic compression of a leg, the compression stocking comprising:
- a first stocking element to be arranged around the lower leg;
- a second stocking element to be arranged around the foot of the leg, wherein the second stocking element is attached to the first stocking element or formed integrally thereon at an angle;
further comprising:
- a pressure compartment to be filled with air up to the adjustable compression pressure and extending in both the first and the second stocking element, and configured to extend all the way around the periphery of at least a part of the lower leg in use in order to exert a homogenous pressure on the at least one part of the lower leg all the way around;
a valve mechanism connected to the pressure compartment, wherein the valve mechanism comprises a closable passage between the pressure compartment and the outside air.

2. Compression stocking according to claim 1, wherein the valve mechanism comprises:
- a valve seat attached to the stocking element;
- a valve lid attached to the valve seat or formed thereon, wherein the passage extends through the valve seat and the valve lid;
- a valve provided in the passage and embodied to be movable between a closing position, in which the valve closes the passage airtightly, and a position allowing passage, in which the valve leaves the passage at least partially open; and
- at least a connecting part between the valve and the valve seat and/or the valve lid for flexible attachment of the valve to the valve seat and/or the valve lid, wherein the at least one connecting part is embodied to urge the valve toward the closing position under bias.

3. Compression stocking according to claim 1 or 2, wherein the pressure compartment wholly surrounds the lower leg in peripheral direction in the situation in which the stocking is being worn so that the lower leg encounters the same pressure all the way around and/or wherein the pressure compartment at the position of the first stocking element extends on the front side, the rear side and on the sides of the lower leg during use and/or wherein the pressure compartment in the first and second stocking element is located over the whole surface of the stocking intended for contact with the leg, with the exception of the surface of the sole and instep.

4. Compression stocking according to any one of the foregoing claims, wherein the pressure is the same in the whole pressure compartment and/or wherein the pressure compartment in the second stocking element extends only on the sides of the foot.

5. Compression stocking according to any one of the foregoing claims, wherein the pressure compartment forms one single, uninterrupted space.

6. Compression stocking according to any one of the foregoing claims, wherein the valve mechanism is connected to the part of the pressure compartment which is situated in the first stocking element.

7. Compression stocking according to any one of the foregoing claims, wherein the compression pressure is smaller than 80 mm Hg and preferably lies between 30 mm Hg and 60 mm Hg.

8. Compression stocking according to any one of the foregoing claims, wherein the second stocking element comprises:
- a sole part;
- an instep part;
- side parts between the sole part and the instep part, wherein the pressure compartment extends in at least one of the side parts, wherein the instep part is preferably manufactured from elastic material and/or wherein the sole part is manufactured from substantially non-elastic material and/or wherein the compression stocking has only one single pressure compartment or wherein the compression stocking has two or more pressure compartments, wherein the pressure compartments are in open connection with each other and/or wherein the pressure compartment extends at least in an area of the second stocking element situated adjacently of the *malleolus lateralis* in a situation of use, and preferably also in a further area adjacently of the *malleolus medialis.*

9. Compression stocking according to any one of the foregoing claims, wherein the at least one connecting part is embodied to be bent between a starting position, in which the valve is in the closing position, and a bent position, in which the valve is in the position allowing passage, under an external mechanical force, wherein in the bent position the connecting part preferably tends to return to the starting position and/or further comprising two or more connecting parts, wherein the valve is disc-like, the connecting parts extend in radial direction relative to the disc-like valve and/or the connecting parts are attached to or formed on the valve at uniformly distributed positions.

10. Compression stocking according to any one of the foregoing claims, in combination with an air pump which is configured to inflate the pressure compartment, wherein the air pump comprises a pump tube or pump conduit which can be coupled to the valve lid and has an outer end which can be inserted into the passage and is embodied to exert on the valve the mechanical force whereby the valve can be pushed from the closing position to the position allowing passage.

11. Compression stocking according to any one of the foregoing claims, comprising a pressure sensor for determining a pressure value representative of the pressure in the pressure compartment, wherein the pressure sensor is preferably embodied to measure the pressure in the pressure compartment and/or wherein the pressure sensor is embodied to measure the pressure in the pump tube or pump conduit, preferably .

12. Compression stocking according to any one of the foregoing claims, comprising a control unit which is coupled to the air pump and pressure sensor and is configured to control the air pump depending on the measured pressure and/or comprising a memory which is coupled to the pressure sensor and is configured to periodically store measured pressure values and/or wherein the pressure sensor comprises a wireless communication unit for wirelessly transmitting a pressure signal representative of the pressure in the pressure compartment, wherein the communication unit, and preferably also the pressure sensor, are optionally configured to be powered only by an external radio frequency signal, wherein the wireless communication unit preferably comprises an RFID transponder.

13. Compression stocking according to any one of the foregoing claims, wherein the stocking element comprises:
- an outer wall with a high modulus of elasticity (E), preferably a modulus of elasticity of at least 50 MPa; and
- a flexible inner wall, directed toward the limb during use, with a low elasticity (E);
and/or wherein the inner wall of the first and/or second stocking element comprises bamboo material and/or wherein the stocking element comprises an outer wall with an elasticity (ε) of a maximum of 5%, preferably a maximum of 2%, when the pressure compartment is inflated to an overpressure of 100 mg Hg.

14. Compression stocking according to any one of the foregoing claims, wherein the stocking element comprises an outer and inner wall manufactured from airtight film, wherein the outer and inner wall are preferably sealed together along their peripheral edges for the purpose of forming the pressure compartment therebetween and/or wherein the stocking element comprises an outer and inner wall manufactured from plastic, particularly thermoplastic polyurethane or polypropylene, and/or wherein the outer wall comprises a wall thickness of between 100 and 300 µm and the inner wall a wall thickness of between 50 and 150 µm.

15. Compression stocking according to any one of the foregoing claims, wherein the outer wall of the stocking element is provided with a number of stretch-limiting elements configured to limit stretch when the pressure compartment is inflated, wherein the stretch-limiting elements preferably extend in peripheral direction when the stocking element is arranged around the limb and/or wherein the first stocking element is manufactured from flexible material which can be wrapped around the leg and/or wherein the stocking element comprises fastening elements with which the first stocking element can be clamped fixedly to the leg.

## Patentansprüche

1. Kompressionsstrumpf mit einstellbarem Kompressionsdruck zur therapeutischen Kompression eines Beines, wobei der Kompressionsstrumpf aufweist:
- ein erstes Strumpfelement, das um den Unterschenkel anzuordnen ist;
- ein zweites Strumpfelement, das um den Fuß des Beins anzuordnen ist, wobei das zweite Strumpfelement an dem ersten Strumpfelement befestigt oder mit diesem unter einem Winkel integral ausgebildet ist;
ferner aufweisend:
- eine Druckkammer, die bis zu dem einstellbaren Kompressionsdruck mit Luft zu füllen ist und sich sowohl in das erste als auch in das zweite Strumpfelement erstreckt, und die so ausgebildet ist, dass sie sich im Gebrauch über den gesamten Umfang zumindest eines Teils des Unterschenkels erstreckt, um einen homogenen Druck auf den zumindest einen Teil des Unterschenkels über dessen gesamten Umfang auszuüben;
- einen Ventilmechanismus, der mit der Druckkammer verbunden ist, wobei der Ventilmechanismus einen verschließbaren Durchgang zwischen der Druckkammer und der Außenluft darstellt.

2. Kompressionsstrumpf nach Anspruch 1, wobei der Ventilmechanismus aufweist:
- einen Ventilsitz, der an dem Strumpfelement befestigt ist;
- einen an dem Ventilsitz angebrachten oder daran angeformten Ventildeckel, wobei sich der Durchgang durch den Ventilsitz und den Ventildeckel erstreckt;
- ein Ventil, das in dem Durchgang vorgesehen und so ausgebildet ist, dass es zwischen einer Schließstellung, in der das Ventil den Durchgang luftdicht verschließt, und einer Durchgangsstellung, in der das Ventil den Durchgang zumindest teilweise offenlässt, bewegt werden kann; und
- zumindest ein Verbindungsteil zwischen dem Ventil und dem Ventilsitz und/oder dem Ventildeckel zur flexiblen Befestigung des Ventils an dem Ventilsitz und/oder dem Ventildeckel, wobei das zumindest eine Verbindungsteil so ausgebildet ist, dass es das Ventil unter Vorspannung in die Schließstellung drängt.

3. Kompressionsstrumpf nach Anspruch 1 oder 2, wobei die Druckkammer den Unterschenkel, wenn der Strumpf getragen wird, in Umfangsrichtung vollständig umgibt, so dass der Unterschenkel rundherum dem gleichen Druck ausgesetzt ist und/oder wobei sich die Druckkammer bei Gebrauch an der Position des ersten Strumpfelements auf der Vorderseite, der Rückseite und an den Seiten des Unterschenkels erstreckt und/oder wobei sich die Druckkammer im ersten und zweiten Strumpfelement über die gesamte für den Kontakt mit dem Bein vorgesehene Oberfläche des Strumpfes erstreckt, mit Ausnahme der Oberflächen von Sohle und Rist.

4. Kompressionsstrumpf nach einem der vorherigen Ansprüche, wobei der Druck in der gesamten Druckkammer gleich ist und/oder wobei sich die Druckkammer im zweiten Strumpfelement nur an den Seiten des Fußes erstreckt.

5. Kompressionsstrumpf nach einem der vorherigen Ansprüche, wobei die Druckkammer einen einzigen, nicht unterbrochenen Raum bildet.

6. Kompressionsstrumpf nach einem der vorherigen Ansprüche, wobei der Ventilmechanismus mit dem Teil der Druckkammer verbunden ist, der sich im ersten Strumpfelement befindet.

7. Kompressionsstrumpf nach einem der vorherigen Ansprüche, wobei der Kompressionsdruck kleiner als 80 mm Hg ist und vorzugsweise zwischen 30 mm Hg und 60 mm Hg liegt.

8. Kompressionsstrumpf nach einem der vorherigen Ansprüche, wobei das zweite Strumpfelement aufweist:
- ein Sohlenteil;
- einen Ristteil;
- zwischen dem Sohlenteil und dem Ristteil angeordnete Seitenteile, wobei sich die Druckkammer in zumindest einem der Seitenteile erstreckt, wobei das Ristteil vorzugsweise aus elastischem Material gefertigt ist, und/oder wobei das Sohlenteil aus im Wesentlichen unelastischem Material gefertigt ist, und/oder wobei der Kompressionsstrumpf nur eine einzige Druckkammer aufweist, oder wobei der Kompressionsstrumpf zwei oder mehr Druckkammern aufweist, wobei die Druckkammern miteinander in offener Verbindung stehen, und/oder wobei sich die Druckkammer bei Gebrauch zumindest in einem zum *Malleolus lateralis* benachbart gelegenen Bereich des zweiten Strumpfelementes und vorzugsweise auch in einem weiteren zum *Malleolus medialis* benachbarten Bereich erstreckt.

9. Kompressionsstrumpf nach einem der vorherigen Ansprüche, wobei das zumindest eine Verbindungsteil so ausgebildet ist, dass es unter einer äußeren mechanischen Kraft zwischen einer Ausgangsposition, in der sich das Ventil in der Schließstellung befindet, und einer gekrümmten Position, in der sich das Ventil in der Durchgangsstellung befindet, gebogen werden kann, wobei das Anschlussteil in der gekrümmten Position vorzugsweise dazu neigt, in die Ausgangsposition zurückzukehren und/oder ferner zwei oder mehr Anschlussteile umfasst, wobei das Ventil scheibenförmig ist, die Anschlussteile sich relativ zu dem scheibenförmigen Ventil in radialer Richtung erstrecken und/oder die Anschlussteile an dem Ventil an gleichmäßig verteilten Positionen befestigt oder daran angeformt sind.

10. Kompressionsstrumpf nach einem der vorherigen Ansprüche in Kombination mit einer zum Aufblasen der Druckkammer ausgebildeten Luftpumpe, wobei die Luftpumpe einen Pumpschlauch oder eine Pumpleitung aufweist, der/die mit dem Ventildeckel gekoppelt werden kann und ein äußeres Ende aufweist, das in den Durchgang eingeführt werden kann und so ausgebildet ist, dass er/sie auf das Ventil jene mechanische Kraft ausübt, durch die das Ventil aus der Schließstellung in die Durchgangsstellung gedrückt werden kann.

11. Kompressionsstrumpf nach einem der vorherigen Ansprüche, aufweisend einen Drucksensor zur Bestimmung eines für den Druck in der Druckkammer repräsentativen Druckwertes, wobei der Drucksensor vorzugsweise zur Messung des Drucks in der Druckkammer ausgebildet ist und/oder wobei der Drucksensor vorzugsweise zur Messung des Drucks im Pumpschlauch oder in der Pumpleitung ausgebildet ist.

12. Kompressionsstrumpf nach einem der vorherigen Ansprüche, aufweisend eine Steuereinheit, die mit der Luftpumpe und dem Drucksensor gekoppelt ist, und dazu konfiguriert ist, die Luftpumpe in Abhängigkeit von dem gemessenen Druck zu steuern, und/oder aufweisend einen Speicher, der mit dem Drucksensor gekoppelt und dazu ausgebildet ist, gemessene Druckwerte periodisch zu speichern, und/oder wobei der Drucksensor eine drahtlose Kommunikationseinheit aufweist, zum drahtlosen Übertragen eines für den Druck in der Druckkammer repräsentativen Drucksignals, wobei die Kommunikationseinheit und vorzugsweise auch der Drucksensor optional dazu ausgebildet sind, lediglich durch ein externes Hochfrequenzsignal mit Energie versorgt zu werden, wobei die drahtlose Kommunikationseinheit vorzugsweise einen RFID-Transponder aufweist.

13. Kompressionsstrumpf nach einem der vorherigen Ansprüche, wobei das Strumpfelement aufweist:
- eine Außenwand mit einem hohen Elastizitätsmodul (E), vorzugsweise einem Elastizitätsmodul von mindestens 50 MPa; und
- eine flexible bei Gebrauch der Extremität zugewandte Innenwand mit einer geringen Elastizität (E);
und/oder wobei die Innenwand des ersten und/oder zweiten Strumpfelements Bambusmaterial umfasst, und/oder wobei das Strumpfelement eine Außenwand aufweist, mit einer Elastizität (ε) von maximal 5 %, vorzugsweise maximal 2 %, wenn die Druckkammer auf einen Überdruck von 100 mg Hg aufgeblasen ist.

14. Kompressionsstrumpf nach einem der vorherigen Ansprüche, wobei das Strumpfelement eine Außen- und eine Innenwand aufweist, welche aus einer luftdichten Folie gefertigt sind, wobei die Außen- und die Innenwand vorzugsweise entlang ihrer Umfangsränder miteinander versiegelt sind, um dazwischen die Druckkammer zu bilden, und/oder wobei das Strumpfelement eine Außen- und eine Innenwand aufweist, welche aus Kunststoff, insbesondere aus thermoplastischem Polyurethan oder Polypropylen, gefertigt sind, und/oder wobei die Außenwand eine Wandstärke zwischen 100 und 300 µm und die Innenwand eine Wandstärke zwischen 50 und 150 µm aufweist.

15. Kompressionsstrumpf nach einem der vorherigen Ansprüche, wobei die Außenwand des Strumpfelements mit einer Anzahl von dehnungsbegrenzenden Elementen versehen ist, die so ausgebildet sind, dass sie, wenn die Druckkammer aufgeblasen wird, die Dehnung begrenzen, wobei sich die dehnungsbegrenzenden Elemente vorzugsweise in Umfangsrichtung erstrecken, wenn das Strumpfelement um die Extremität herum angeordnet ist, und/oder wobei das erste Strumpfelement aus flexiblem Material gefertigt ist, welches um das Bein gewickelt werden kann, und/oder wobei das Strumpfelement Befestigungselemente aufweist, mit denen das erste Strumpfelement fest an das Bein angeordnet werden kann.

## Revendications

1. Bas de contention à pression de compression réglable pour la compression thérapeutique d'une jambe, le bas de contention comprenant :
- un premier élément de bas à agencer autour de la partie inférieure de jambe ;
- un deuxième élément de bas à agencer autour du pied de la jambe, dans lequel le deuxième élément de bas est fixé au premier élément de bas ou formé d'un seul tenant sur celui-ci à un angle ;
comprenant en outre :
- un compartiment de pression à remplir d'air jusqu'à la pression de compression réglable, s'étendant à la fois dans le premier et le deuxième élément de bas, et configuré pour s'étendre tout autour de la périphérie d'au moins une partie de la partie inférieure de jambe en cours d'utilisation afin d'exercer une pression homogène sur l' au moins une partie de la partie inférieure de jambe tout autour de celle-ci ;
un mécanisme de soupape connecté au compartiment de pression, dans lequel le mécanisme de soupape comprend un passage pouvant être fermé entre le compartiment de pression et l'air extérieur.

2. Bas de contention selon la revendication 1, dans lequel le mécanisme de soupape comprend :
- un siège de soupape fixé à l'élément de bas ;
- un couvercle de soupape fixé au siège de soupape ou formé sur celui-ci, dans lequel le passage s'étend à travers le siège de soupape et le couvercle de soupape ;
- une soupape prévue dans le passage et conçue pour être mobile entre une position de fermeture dans laquelle la soupape ferme le passage de manière étanche à l'air, et une position permettant le passage dans laquelle la soupape laisse le passage au moins partiellement ouvert ; et
- au moins une partie de connexion entre la soupape et le siège de soupape et/ou le couvercle de soupape pour une fixation souple de la soupape au siège de soupape et/ou au couvercle de soupape, dans lequel l'au moins une partie de connexion est conçue pour pousser la soupape vers la position de fermeture sous l'effet d'une sollicitation.

3. Bas de contention selon la revendication 1 ou 2, dans lequel dans la situation où le bas est porté, le compartiment de pression entoure entièrement la partie inférieure de jambe dans une direction périphérique de sorte à ce que la partie inférieure de jambe subisse la même pression tout autour de celle-ci, et/ou dans lequel à la position du premier élément de bas, le compartiment de pression s'étend sur le côté avant, le côté arrière et les côtés de la partie inférieure de jambe pendant l'utilisation, et/ou dans lequel le compartiment de pression dans les premier et deuxième éléments de bas est situé sur toute la surface du bas destinée à être en contact avec la jambe, à l'exception de la surface de la semelle et du cou-de-pied.

4. Bas de contention selon l'une quelconque des revendications précédentes, dans lequel la pression est la même dans tout le compartiment de pression, et/ou dans lequel le compartiment de pression dans le deuxième élément de bas s'étend uniquement sur les côtés du pied.

5. Bas de contention selon l'une quelconque des revendications précédentes, dans lequel le compartiment de pression forme un seul espace ininterrompu.

6. Bas de contention selon l'une quelconque des revendications précédentes, dans lequel le mécanisme de soupape est connecté à la partie du compartiment de pression qui est située dans le premier élément de bas.

7. Bas de contention selon l'une quelconque des revendications précédentes, dans lequel la pression de compression est inférieure à 80 mm Hg et se situe de préférence entre 30 mm Hg et 60 mm Hg.

8. Bas de contention selon l'une quelconque des revendications précédentes, dans lequel le deuxième élément de bas comprend :
- une partie semelle ;
- une partie cou-de-pied ;
- des parties latérales entre la partie semelle et la partie cou-de-pied, dans lequel le compartiment de pression s'étend dans au moins une des parties latérales, dans lequel la partie cou-de-pied est de préférence fabriquée à partir d'un matériau élastique, et/ou dans lequel la partie semelle est fabriquée à partir d'un matériau sensiblement non élastique, et/ou dans lequel le bas de contention a uniquement un seul compartiment de pression ou dans lequel le bas de contention a deux compartiments de pression ou plus, dans lequel les compartiments de pression sont en connexion ouverte l'un avec l'autre, et/ou dans lequel le compartiment de pression s'étend au moins dans une zone du deuxième élément de bas située de manière adjacente de la *malléole latérale* dans une situation d'utilisation et de préférence également dans une zone supplémentaire de manière adjacente de la *malléole médiale.*

9. Bas de contention selon l'une quelconque des revendications précédentes, dans lequel l' au moins une partie de connexion est conçue pour être pliée entre une position de départ dans laquelle la soupape est dans la position de fermeture et une position pliée dans laquelle la soupape est dans la position permettant le passage sous l'effet d'une force mécanique externe, dans lequel dans la position pliée, la partie de connexion tend de préférence à revenir à la position de départ et/ou comprend en outre deux parties de connexion ou plus, dans lequel la soupape est en forme de disque, les parties de connexion s'étendent dans une direction radiale par rapport à la soupape en forme de disque et/ou les parties de connexion sont fixées à la soupape ou formées sur celle-ci à des positions uniformément réparties.

10. Bas de contention selon l'une quelconque des revendications précédentes, en combinaison avec une pompe à air qui est configurée pour gonfler le compartiment de pression, dans lequel la pompe à air comprend un tube de pompe ou un conduit de pompe qui peut être couplé au couvercle de soupape et a une extrémité extérieure qui peut être insérée dans le passage et qui est conçue pour exercer la force mécanique sur la soupape, ce qui permet de pousser la soupape de la position de fermeture à la position permettant le passage.

11. Bas de contention selon l'une quelconque des revendications précédentes, comprenant un capteur de pression pour déterminer une valeur de pression représentative de la pression dans le compartiment de pression, dans lequel le capteur de pression est de préférence conçu pour mesurer la pression dans le compartiment de pression, et/ou dans lequel le capteur de pression est conçu pour mesurer la pression dans le tube de pompe ou le conduit de pompe, de préférence.

12. Bas de contention selon l'une quelconque des revendications précédentes, comprenant une unité de commande qui est couplée à la pompe à air et au capteur de pression et qui est configurée pour commander la pompe à air en fonction de la pression mesurée, et/ou comprenant une mémoire qui est couplée au capteur de pression et qui est configurée pour stocker périodiquement des valeurs de pression mesurées, et/ou dans lequel le capteur de pression comprend une unité de communication sans fil pour transmettre sans fil un signal de pression représentatif de la pression dans le compartiment de pression, dans lequel l'unité de communication, et de préférence également le capteur de pression, sont facultativement configurés pour être alimentés uniquement par un signal de radiofréquence externe, dans lequel l'unité de communication sans fil comprend de préférence un transpondeur RFID.

13. Bas de contention selon l'une quelconque des revendications précédentes, dans lequel l'élément de bas comprend :
- une paroi extérieure à module d'élasticité élevée (E), de préférence un module d'élasticité d'au moins 50 MPa ; et
- une paroi intérieure souple dirigée vers le membre pendant l'utilisation, à faible élasticité (E) ;
et/ou dans lequel la paroi intérieure du premier et/ou du deuxième élément de bas comprend un matériau en bambou, et/ou dans lequel l'élément de bas comprend une paroi extérieure d'une élasticité (ε) de 5 % maximum, de préférence de 2 % maximum, lorsque le compartiment de pression est gonflé à une surpression de 100 mg Hg.

14. Bas de contention selon l'une quelconque des revendications précédentes, dans lequel l'élément de bas comprend des parois extérieure et intérieure fabriquées à partir d'un film étanche à l'air, dans lequel les parois extérieure et intérieure sont de préférence scellées l'une à l'autre le long de leurs bords périphériques afin de former le compartiment de pression entre elles, et/ou dans lequel l'élément de bas comprend des parois extérieure et intérieure fabriquées en plastique, en particulier le polyuréthane thermoplastique ou le polypropylène, et/ou dans lequel la paroi extérieure comprend une épaisseur de paroi entre 100 et 300 µm, et la paroi intérieure comprend une épaisseur de paroi de entre 50 et 150 µm.

15. Bas de contention selon l'une quelconque des revendications précédentes, dans lequel la paroi extérieure de l'élément de bas est dotée d'un certain nombre d'éléments limitant l'étirement configurés pour limiter l'étirement lorsque le compartiment de pression est gonflé, dans lequel les éléments limitant l'étirement s'étendent de préférence dans une direction périphérique lorsque l'élément de bas est agencé autour du membre, et/ou dans lequel le premier élément de bas est fabriqué à partir d'un matériau souple qui peut être enroulé autour de la jambe, et/ou dans lequel l'élément de bas comprend des éléments de fixation avec lesquels le premier élément de bas peut être serré de manière fixe à la jambe.
